# EUROPEAN PATENT APPLICATION

(11) **EP 1 837 037 A1**
(43) Date of publication of application: **26.09.2007**
(21) Application number: 07104827.6
(22) Date of filing: 23.03.2007
(51) Int. Cl.: A61L 2/20, A61L 2/22

(54) **Method and apparatus for sterilizing containers**

(30) Priority: 23.03.2006 IT PR20060024
(71) Applicant: PROCOMAC S.p.A., 43038 Sala Baganza (Parma) (IT)
(72) Inventor: FERRAGUTI, Pietro, c/o PROCOMAC S.p.A., 43100 Parma (IT)
(74) Representative: Gotra, Stefano

(57) **Abstract**

A method for sterilising containers, comprising the steps of atomising a sterilising liquid; mixing the atomised liquid with a carrier fluid to obtain a sterilising mixture, said carrier fluid having such a temperature as to make the atomised liquid evaporate; sterilising a container by sending said sterilising mixture against inner and/or outer walls of the container. During the sterilisation step, the temperature of the sterilising mixture is maintained above a condensation temperature of the sterilising liquid. Apparatus (1) for sterilising containers (100).

## Description

The present invention relates to a method for sterilising containers of the type described in the preamble to claim 1.

The present invention further relates to a related apparatus for sterilising containers, as described in the preamble to claim 16.

As is well known, in the food industry, in particular in the field of the aseptic filling of containers with drinks and in the field of the packaging of foods, also in aseptic conditions, there is the need to sterilise the containers internally and externally, before they are filled.

Generally, the sterilisation is conducted with chemical agents, such as hydrogen peroxide or peracetic acid, which are usable on surfaces of every kind, such as paper, plastic, metal or inorganic materials.

In accordance with a first prior art described in the documents US 4,742,667 and

US 4,631,173, there is a sterilisation method in which the sterilising mixture, constituted by a sterilising liquid mixed with a gas with inert behaviour with respect to the liquid, is heated to obtain a vapour/inert gas mixture that is immediately sent against the object to be sterilised. As a result of the contact with the surfaces of the object that are at a lower temperature than a condensation temperature of the sterilising liquid, the vapour present in the mixture condenses.

In accordance with a second prior art, the document US 6,596,231 discloses a method for hyper-activating sterilisation fluids that uses a sterilising fluid mixed with a gas that is inert with respect to said sterilising fluid, to form a sterilising mixture.

In particular, the sterilising mixture thus obtained is made to evaporate within an evaporation tube under annular motion conditions; specifically, the liquid phase of the mixture flows in contact with the heated walls of the evaporator at a slower speed than the speed of the gaseous phase, which, instead, flows in the central area of the evaporator, within the liquid phase.

A vapour/inert-gas mixture is thereby obtained which is sent to a sterilisation chamber, in which the temperature is lower than a condensation temperature of the sterilising fluid.

Specifically, the vapour present in the gaseous mixture condensates on the product to be sterilised, after the mixture is sent against the product.

Both prior art techniques described above comprise a step of rinsing the containers with sterile hot water, to remove the residues of sterilising liquid at the end of the sterilisation step.

The methods described above have some important drawbacks.

First of all, the need to rinse the containers with sterile hot water to remove the residues of condensed vapour on the object to be sterilised considerably limits the efficiency of a sterilisation plant, because it slows its production cycle.

An additional advantage of the methods in accordance with the prior art is represented by the difficulty of removing the residues of sterilising liquid from the container, after the liquid has carried out is sterilising action. The sterile hot water used to remove the sterilising liquid from the container cannot be made to flow out easily and therefore it is necessary to perform a step of draining and drying the containers, which further slows the production cycle.

An object of the present invention is to eliminate the aforementioned drawbacks, making available a method for sterilising containers that allows an immediate evacuation of the sterilising substance, after it carries out its sterilising action.

Another object of the present invention is to propose a method for sterilising containers that does not require a step of rinsing with sterile hot water.

An additional object of the present invention is to make available a method for sterilising containers that does not require any step of drying the containers.

A further object of the present invention is to propose a method that is particularly suited for sterilising containers made with materials that have porosity, such as bottles made of PET.

An additional object of the present invention is to provide a sterilising apparatus that is able to implement a method for sterilising containers in accordance with the invention.

Yet another object of the present invention is to propose an apparatus and a method that are able to prevent any uncontrolled deformation of the container during the sterilisation operation.

In particular, an object of the present invention is to provide a sterilisation apparatus that is reliable and easy to construct.

Said objects are fully achieved by the method for sterilising containers and by the related apparatus of the present invention, which are characterised by the content of the claims set out below:

This and other objects shall become more readily apparent in the description that follows of a preferred embodiment, illustrated purely by way of non limiting example in the accompanying drawing tables, in which:
- figure 1 shows a global view of an apparatus for sterilising containers according to the present invention;

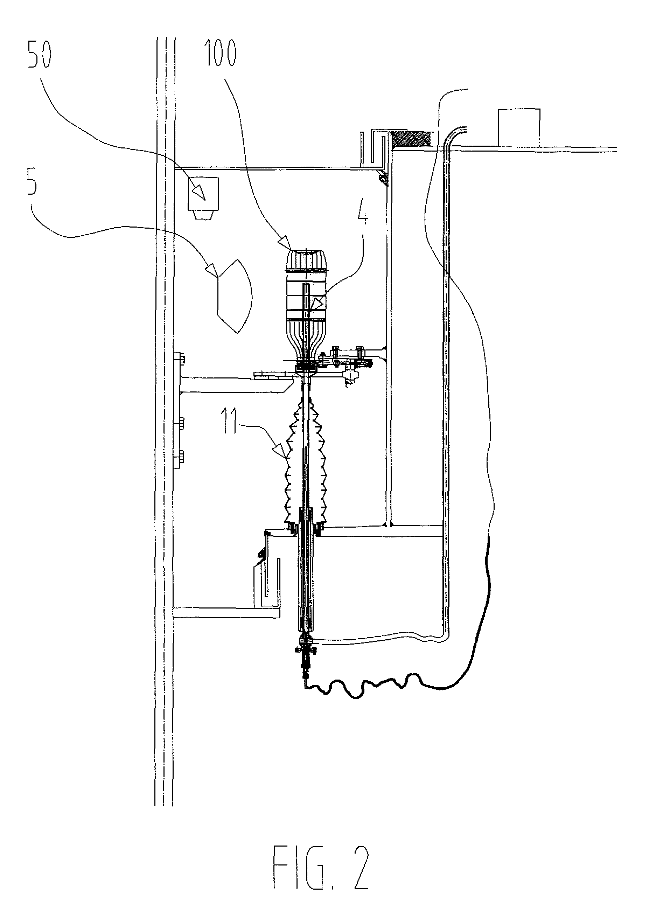

- figure 2 shows an enlarged lateral view of a part of the apparatus shown in Figure 1;
- figure 3a shows a lateral view of a possible embodiment variant of a constructive detail of the apparatus shown in figures 1 and 2;
- figure 3b shows an enlarged lateral view of a constructive detail of the apparatus shown in figure 1.

A method for sterilising containers, in particular bottles made of PET, according to the present invention comprises the steps of:
- atomising a sterilising liquid;
- mixing the atomised liquid with a carrier fluid to obtain a sterilising mixture, said carrier fluid having such a temperature as to make the atomised liquid evaporate;
- sterilising a container by sending said sterilising mixture against inner and outer walls of the container itself.

In new and original fashion with respect to prior art methods, the method according to the invention provides that, during the sterilisation, the temperature of the sterilising mixture is maintained above a condensation temperature of the sterilising liquid.

In the preferred embodiment, the sterilising liquid is hydrogen peroxide, whilst the carrier fluid is preferably hot, dry air that is mixed with the atomised liquid at a temperature ranging between about 60 and 120°C, at a pressure typically lower than 0.5 bar and at such a relative humidity as to maintain a concentration of the sterilising mixture above 2000 ppm.

The condensation temperature of the sterilising liquid within the sterilising mixture depends on the pressure with which the mixture itself is sent against the inner and/or outer walls of the container to be sterilised. Depending on the pressure present inside and/or outside the containers, the temperature at which the sterilising mixture is to be maintained during the sterilisation operation will be selected on the basis of the known phase diagrams of the substances used as sterilising agents.

The method comprises a step of heating the container to be sterilised to maintain it at a temperature higher than a condensation temperature of the sterilising liquid.

Maintaining the walls of the container at a higher temperature than the condensation temperature of the sterilising liquid, said liquid does not undergo a vapour/liquid phase change after impacting the walls of the container, contrary to what takes place in methods according to the prior art.

In particular, the step of heating the containers is preferably carried out by irradiation by means of electric resistors whereto the containers are approached, before and/or during the sterilisation step.

Heating the containers, in addition to preventing the condensation of the vapour on the walls of the containers, heats the air present inside the containers, preventing the condensation of the vapour inside the containers.

With reference to containers made of PET, the step of heating the containers is conducted at a temperature between about 45°C and about 65°C. If containers made of glass are treated, markedly higher heating temperatures can be used, up to about 150°C.

The method comprises a step of controlling the temperature of the containers. In particular, said temperature control step preferably takes place by means of an infrared sensor which is maintained in proximity of the containers to be sterilised, before and/or during said sterilisation step.

The method further comprises a step of heating means able to send the sterilising mixture against inner and/or outer walls of a container to maintain the sterilising mixture at a lower temperature than a condensation temperature of the sterilising liquid, to prevent the condensation of the sterilising liquid, during the step of sending the mixture. In the preferred embodiment, said step of heating means able to send the sterilising mixture is performed at a temperature which may vary according to the containers to be treated and included between 45°C and about 120°C.

Preferably, said means able to send the mixture against inner and/or outer walls of the container comprising one or heated nozzles. In particular, if it is necessary to sterilise the inner walls, the nozzle is inserted into the container and, by effect of the heating whereto it is subjected, it contributes to heat the air present within the containers, preventing the condensation of the vapour. In particular, the heating of the air inside the containers takes place mainly by heat conduction and convention and partly also by irradiation.

The method according to the invention further comprises a step of controlling the temperature of the means for sending the sterilising mixture. In particular, said step of controlling the temperature is preferably carried out by means of a thermocouple or a thermo-resistor associated to the means for sending the sterilising mixture.

In the preferred embodiment, the sterilising liquid is contained in a chamber provided with atomising means. In particular, although said atomising means can include various types of mechanical, pneumatic or piezoelectric atomisers, are preferably obtained by means of a piezoelectric crystal which is excited electrically to vibrate at a frequency of about 2 MHz and cause the vaporisation of the sterilising liquid.

The method also comprises a final step of sending air to remove any residues of sterilising mixture from inner and/or outer walls of a container, after the sterilisation step. In particular, the temperature of the air is variable according to the container to be treated and it ranges between about 45°C and about 150°C. Said step of sending air is necessary to inactivate the sterilising agent remaining in the container.

Essentially, to prevent condensation during the sterilisation step, the sterilising liquid is maintained at a temperature higher than a condensation temperature, first by the heat that it receives from the carrier fluid and subsequently by the heat that it receives from the means able to send the mixture against the walls of a container to be sterilised, which, as stated, are heated and/or by the heating of the containers.

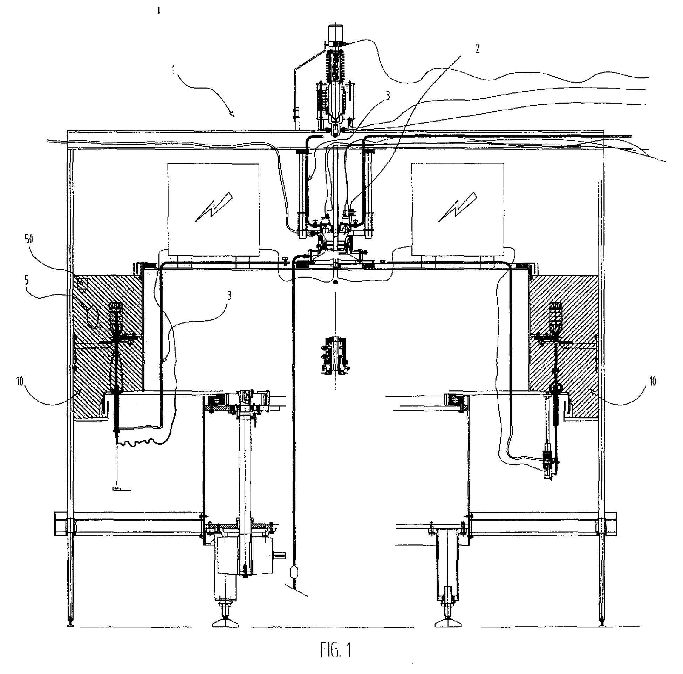

With particular reference to figure 1, an apparatus for sterilising containers 100 able to implement a sterilisation method according to the present invention is globally designated by the number 1.

The apparatus 1 comprises a chamber 2 for a sterilising liquid and means for atomising said sterilising liquid in a carrier fluid to form a sterilising mixture.

In the preferred embodiment, the means for atomising the sterilising liquid (not shown) are piezoelectric and they preferably comprise a piezoelectric crystal inserted in the chamber 2.

Preferably, said piezoelectric crystal is excited electrically to vibrate at a frequency of about 2 MHz and to cause the vaporisation of the sterilising liquid.

The apparatus 1 comprises means for sending the carrier fluid, preferably comprising air, at an outlet of the chamber 2, wherefrom outflows the sterilising liquid already atomised. Preferably, said means are constituted by channels 3.

In this way, the atomised liquid is mixed with the carrier fluid to form a sterilising mixture.

In the preferred embodiment, the carrier fluid is pre-heated to a temperature such as to cause the atomised liquid to evaporate after the mixing. The preheating takes place before the carrier fluid is sent at an outlet of the chamber 2.

In the preferred embodiment, the sterilising liquid is hydrogen peroxide, whilst the carrier fluid is preferably hot, dry air that is mixed with the atomised liquid at a temperature ranging between about 60°C and 120°C, at a pressure typically lower than 0.5 bar and at such a relative humidity as to maintain a concentration of the sterilising mixture above 2000ppm.

The apparatus 1 is provided with means for sending the sterilising mixture into a container. In particular, said means for sending the sterilising mixture into the container preferably comprise a tubular nozzle 4 movable between a first operative configuration, in which the nozzle is completely external to the container, and a second operative configuration, in which the nozzle is at least partially inserted inside the container. Preferably, the nozzle 4 is covered by a bellows 11.

In novel and original fashion, the apparatus 1 comprises means for heating the nozzle, to maintain it at a temperature above the condensation temperature of the sterilising liquid.

In the preferred embodiment, said heating means comprise electrical resistors (not shown) fastened on the nozzle 4.

The apparatus 1 is also provided with means for controlling the temperature of the nozzle that co-operate with said heating means.

Preferably, the means for controlling the temperature of the nozzle comprise at least an internal sensor (not shown) and a typically, but not exclusively, infrared external sensor 5 .

Advantageously, the presence of electrical resistors and temperature sensors assures reliability and traceability to the system.

In accordance with an embodiment variant, said internal sensor for controlling the temperature comprises at least one thermocouple or a thermo-resistance.

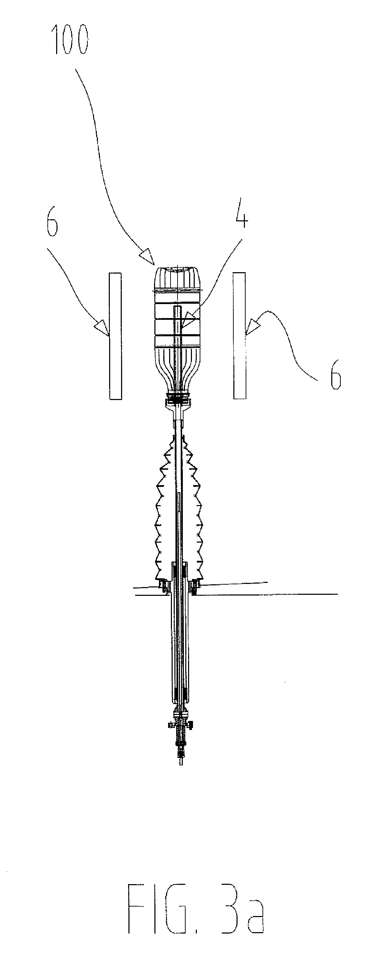

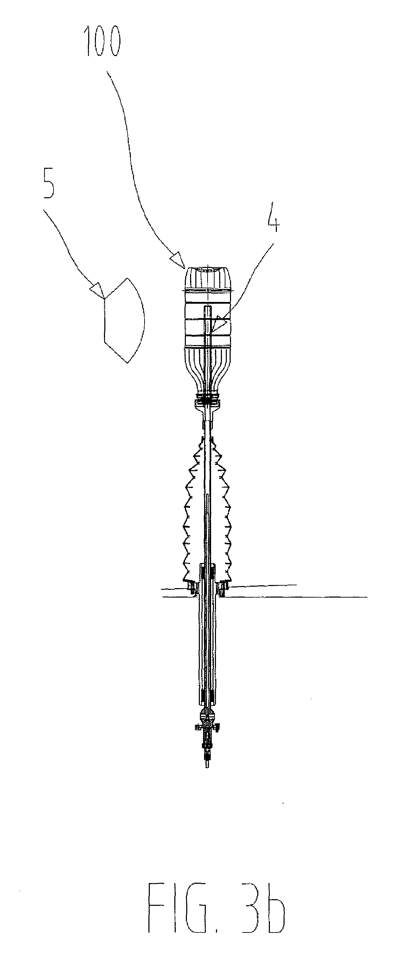

The apparatus 1 is also provided means for heating the containers to be sterilised. Preferably, said means for heating the containers are obtained by means of a pair of plates 6 incorporating one or more electrical resistors and positioned outside the sterilisation chambers 10. In particular, said plates 6 face each other and they are approached to the containers in such a way as to operate heating thereof by irradiation, preferably before the containers are transferred into the sterilisation chambers 10 by means of a transfer carrousel or other known devices, not shown. According to the embodiment variant shown in Figure 3a, the plates 6 can be positioned inside the sterilisation chambers 10, in such a way as to heat a container when the nozzle 4 is already inserted within it.

With reference to containers made of PET, the heating of the containers is conducted at a temperature between about 45°C and about 65°C. If containers made of glass are treated, markedly higher heating temperatures can be used, up to about 150°C.

The apparatus 1 also comprises means for sending into the container flow of air having a temperature that is variable according to the container to be treated and anyway ranges between about 45°C and about 150°C and an extremely low relative humidity, depending on the input conditions. Said flow of air is able to remove any residues of sterilising mixture from the walls of the treated container.

The apparatus 1 further comprises a sensor 50 for monitoring the concentration of the sterilising liquid within the sterilising mixture and in particular in the sterilisation chambers 10, to allow the traceability of the system, i.e. to know precisely in what way the sterilisation treatment was conducted on the container. The invention achieves important advantages.

First of all, a method for sterilising containers according to the invention allows an immediate evacuation of the sterilising mixture, after said mixture has completed its sterilising action. Since the sterilising mixture is in the gaseous state throughout the sterilisation step, no step of draining the containers is necessary, unlike in methods in accordance with the prior art.

In the second place, a method according to the invention requires no step of rinsing with sterile hot water and therefore it is not necessary to carry out any step of draining or drying the containers.

Advantageously, a method and an apparatus according to the invention are particularly suited for sterilising containers made with materials that have porosity, such as bottles made of PET.

Another advantage of a method according to the invention is the ability to prevent any uncontrolled deformation of the container during the sterilisation operation.

Advantageously, the presence of a first step of heating the sterilising mixture to such a temperature as not to have a potential able to deform the containers and of a second step of heating to a higher temperature directly controlled on the nozzle enable to conduct a sterilisation under optimal conditions, with the possibility of interruption during transients and machine idle times, to avoid any uncontrolled deformation of the container.

## Claims

1. Method for sterilising containers, comprising the steps of:
atomising a sterilising liquid;
mixing the atomised liquid with a carrier fluid to obtain a sterilising mixture, said carrier fluid having such a temperature as to make the atomised liquid evaporate;
sterilising a container by sending said sterilising mixture against inner and/or outer walls of the container itself,
**characterised in that**, during said sterilisation step, the temperature of the sterilising mixture is maintained above a condensation temperature of the sterilising liquid.

2. A method as claimed in claim 1, **characterised in that** it comprises a step of heating the container to be sterilised to maintain the sterilising mixture at a temperature higher than a condensation temperature of the sterilising liquid.

3. A method as claimed in claim 2, **characterised in that** said step of heating the container is conducted at a temperature between about 45 °C and 150 °C.

4. A method as claimed in any one of the claims 2 or 3, **characterised in that** it comprises a step of controlling the temperature of the container.

5. A method as claimed in claim 4, **characterised in that** said step of controlling the temperature of the container takes place by means of an infrared sensor.

6. A method as claimed in any of the previous claims, **characterised in that** it comprises a step of heating means suitable for sending the sterilising mixture against inner and/or outer walls of a container to maintain the sterilising mixture at a higher temperature than a condensation temperature of the sterilising liquid.

7. A method as claimed in claim 6, **characterised in that** said step of heating means able to send the sterilising mixture against inner and/or outer walls of a container is conducted at a temperature between about 45 °C and 150 °C.

8. A method as claimed in claims 6 or 7, **characterised in that** it comprises a step of controlling the temperature of said means able to send the sterilising mixture against inner and/or outer walls of a container.

9. A method as claimed in claim 1, **characterised in that** said carrier fluid comprises air.

10. A method as claimed in any of the previous claims, **characterised in that** the sterilising liquid is contained in a chamber provided with atomising means.

11. A method as claimed in claim 10, **characterised in that** said atomising means are piezoelectric.

12. A method as claimed in claim 10 or 11, **characterised in that** said atomising means comprise at least one piezoelectric crystal.

13. A method as claimed in any of the previous claims, **characterised in that** said sterilising liquid comprises hydrogen peroxide.

14. A method as claimed in any of the previous claims, **characterised in that** it comprises a step of sending air at a temperature between about 45°C and 150°C to remove any residues of sterilising mixture from inner and/or outer walls of a container, after said sterilisation step.

15. A method as claimed in claim 3, **characterised in that** said step of heating the container takes place by irradiation.

16. An apparatus (1) for sterilising containers (100), comprising:
a chamber (2) for a sterilising liquid having at least one outlet;
means (3) for sending a carrier fluid at said outlet of the chamber (2);
means for atomising said sterilising liquid in the carrier fluid to form a sterilising mixture, said carrier fluid having such a temperature as to make the atomised liquid evaporate;
means (4) for sending said sterilising mixture into a container (100),
**characterised in that** it comprises heating means operatively active on said means (4) for sending the sterilising mixture into a container to maintain said means at a temperature higher than a condensation temperature of the sterilising liquid.

17. An apparatus as claimed in claim 16, **characterised in that** said means for sending the sterilising mixture into the container comprise a tubular body (4) movable between a first operative configuration, in which the tubular body (4) is completely external to the container (100), and a second operative configuration, in which the tubular body (4) is at least partially inserted inside the container (100), said tubular body (4) having an outlet for dispensing a pre-set quantity of sterilising mixture.

18. Apparatus as claimed in claim 16, **characterised in that** said heating means comprise electrical resistors.

19. An apparatus as claimed in any of the previous claims 16 through 18, **characterised in that** it comprises means for controlling the temperature of said means (4) for sending the sterilising mixture into a container (100), said means for controlling the temperature co-operating with said heating means.

20. An apparatus as claimed in claim 19, **characterised in that** said means for controlling temperature comprise at least one infrared sensor (5).

21. An apparatus as claimed in claim 19, **characterised in that** said means for controlling the temperature comprise at least one thermocouple and one thermo-resistor.

22. An apparatus as claimed in any of the previous claims 16 through 21, **characterised in that** said means for atomising the sterilising liquid are piezoelectric.

23. An apparatus as claimed in claim 21 or 22, **characterised in that** said means for atomising the sterilising liquid comprise at least one piezoelectric crystal operatively associated with said chamber (3) and immersed in the liquid.

24. An apparatus as claimed in any of the previous claims 16 through 23, **characterised in that** it comprise means for heating the containers (100) to be sterilised.

25. An apparatus as claimed in claim 24, **characterised in that** said means for heating the containers to be sterilised comprise at least one electric resistor.

26. An apparatus as claimed in any of the previous claims from 16 through 25, **characterised in that** it comprises means for sending into the container (100) a flow of air having a temperature between about 45°C and about 150°C to remove any residues of sterilising mixture.

27. An apparatus as claimed in any of the previous claims from 1 through 26, **characterised in that** it comprises means for monitoring the concentration of the sterilising liquid within the sterilising mixture.
